(19) **Europäisches Patentamt**
**European Patent Office**
**Office européen des brevets**

(11) **EP 1 691 709 B1**

(12) **EUROPEAN PATENT SPECIFICATION**

(45) Date of publication and mention
of the grant of the patent:
**27.08.2008 Bulletin 2008/35**

(51) Int Cl.:
***A61B 19/04*** *(2006.01)* ***A61F 6/04*** *(2006.01)*

(21) Application number: **04777614.1**

(86) International application number:
**PCT/US2004/021612**

(22) Date of filing: **06.07.2004**

(87) International publication number:
**WO 2005/060856 (07.07.2005 Gazette 2005/27)**

(54) **METHOD FOR FORMING AN ELASTOMERIC ARTICLE**

VERFAHREN ZUR BILDUNG EINES ELASTOMEREN ARTIKELS

PROCEDE DE FORMATION D'ARTICLE ELASTOMERE

(84) Designated Contracting States:
**DE FR GB IT NL**

(30) Priority: **11.12.2003 US 733155**

(43) Date of publication of application:
**23.08.2006 Bulletin 2006/34**

(73) Proprietor: **KIMBERLY-CLARK WORLDWIDE, INC.**
**Neenah, Wisconsin 54956 (US)**

(72) Inventors:
 • **MODHA, Shantilal, Hirji**
 **Alpharetta, GA 30004 (US)**
 • **KISTER, Mary, Elizabeth**
 **Cumming, GA 30040 (US)**

 • **NGUYEN, KC**
 **Neenah, WI 54957-0349 (US)**

(74) Representative: **Davies, Christopher Robert**
**Frank B. Dehn & Co.**
**St Bride's House**
**10 Salisbury Square**
**London**
**EC4Y 8JD (GB)**

(56) References cited:
 **WO-A-02/32475** **WO-A-99/19006**
 **CA-A- 2 259 715** **US-A- 4 499 154**
 **US-A- 5 300 059** **US-A- 5 817 365**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Background of the Invention**

Elastomeric gloves, such as surgical and examination gloves, have traditionally been made of natural or synthetic elastomers to provide a combination of good elasticity and strength. To form a natural rubber glove, for instance, a hand-shaped former is initially dipped into a coagulant composition and subsequently into a natural rubber latex bath. The coagulant causes the latex to deposit on the former. The coagulant composition may include a salt, such as calcium carbonate or calcium nitrate, which facilitates the formation of an elastomeric polymer into a substrate body and aids in removal of the tacky polymer from the former. Surfactants may also be used in the coagulant composition. US 4,499,154,

WO 02/32475, disclose prior art methods for forming elastomeric gloves in which the application of a surfacant is applied by tumbling once the glove has been stripped from the former >

Once formed, the latex substrate body is then subjected to various treatment processes to improve its properties. For example, due to the tight fit of elastomeric gloves over the hand, they are often difficult to don. As a result, various techniques have been developed to aid in the donnability of elastomeric gloves. Many conventional treatment steps require the glove to be stripped from the former used in the "on-line" glove-forming process, and sent "off-line" for the desired post-treatment. For example, gloves are often lubricated off-line in a tumbler. To strip the glove from the former for such off-line processing, it is typically necessary to apply an antiblocking powder to the outer surface of the substrate body (facing away from the former), such as calcium carbonate, calcium stearate, magnesium carbonate, and so forth, which inhibits the glove from sticking to itself when stripped. For example, prior to stripping, the former may be dipped into a slurry that contains the antiblocking powder. Unfortunately, the use of such powders has many drawbacks in the manufacture of some types of gloves, such as those used in surgical or clean room environments. For example, powder may enter a wound and cause complications for the patient. Powder may also carry infectious agents and/or cause allergenic reactions in the patient. Thus, several techniques have been developed to remove antiblocking powders from gloves. For example, gloves may be chlorinated "off-line" using well-known techniques (e.g., a chlorinator) to remove the antiblocking powder prior to use of the glove.

Despite the benefits achieved using conventional glove-forming processes, a need for improvement nevertheless remains. Specifically, the requirement of various "off-line" post-treatment steps is time-consuming, costly, and inefficient. Thus, a need continues to remain for a method of forming a glove in a more efficient and cost-effective manner.

**Summary of the Invention**

In accordance with the present invention, a method for forming an elastomeric glove is disclosed, as set forth is claim 1.

Other features and aspects of the present invention are discussed in greater detail below.

**Brief Description of the Drawings**

A full and enabling disclosure of the present invention, including the best mode thereof, directed to one of ordinary skill in the art, is set forth more particularly in the remainder of the specification, which makes reference to the appended figures in which:

Fig. 1 is a perspective view of one embodiment of an elastomeric glove made according to the invention; and
Fig. 2 is a cross-sectional view of the glove illustrated in Fig. 1 taken along a line 2-2.

Repeat use of reference characters in the present specification and drawings is intended to represent same or analogous features or elements of the invention.

**Detailed Description of Representative Embodiments**

Reference now will be made in detail to various embodiments of the invention, one or more examples of which are set forth below. Each example is provided by way of explanation of the invention, not limitation of the invention. In fact, it will be apparent to those skilled in the art that various modifications and variations may be made in the present invention without departing from the scope of the claims. For instance, features illustrated or described as part of one embodiment, may be used on another embodiment to yield a still further embodiment. Thus, it is intended that the present invention covers such modifications and variations as come within the scope of the appended claims.

In general, the present invention is directed to an elastomeric article (e.g., glove, condom, and so forth) that is coated with a hydrogel. The hydrogel coating facilitates dry and/or damp donning. In addition, due its low coefficient of friction, the hydrogel allows stripping of a dip-formed article without the use of an antiblocking powder. Thus, the present inventors have discovered that certain treatment steps, such as chlorination and/or lubrication, conventionally conducted "off-line" (i.e., after stripping) are no longer required. Moreover, even when such treatments are used, they may be conducted "on-line" (i.e., before stripping). The ability to eliminate certain off-line treatment steps provides a significant improvement in the efficiency of the forming process.

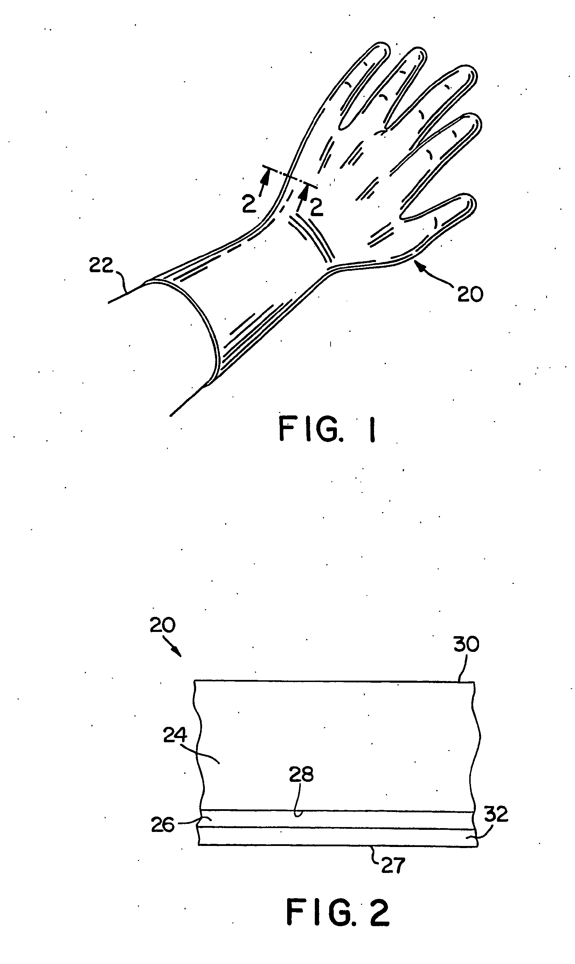

Referring to Figs. 1-2, for example, one embodiment of an elastomeric glove 20 is illustrated that may be placed on the hand of a user 22. The glove 20 includes a substrate body 24 having the basic shape of the glove, e.g., having an inner surface and an outer surface that define a hand-shaped cavity. The substrate body 24 is generally formed from an emulsion-based elastomeric material (e.g., polymers formed by emulsion polymerization). Some examples of materials that be used to form the emulsion-based elastomeric material include, but are not limited to, natural rubber latex, isoprene polymers, chloroprene polymers, vinyl chloride polymers, butadiene polymers, styrene-butadiene polymers, carboxylated styrene-butadiene polymers, acrylonitrile-butadiene polymers, carboxylated acrylonitrile-butadiene polymers, acrylonitrile-styrene-butadiene polymers, carboxylated acrylonitrile-styrene-butadiene polymers, derivatives thereof, and so forth. Combinations of elastomeric materials may also be used in a single layer of an article or in separate layers, such as in a multi-layer article.

In one embodiment, the substrate body 24 is formed from natural rubber latex. To form the substrate body 24 from natural latex, a former is initially dipped into a coagulant bath that facilitates later stripping of the glove from the former. The coagulant bath may include calcium carbonate and/or calcium nitrate. Thereafter, the coagulant-coated former is dried and subsequently dipped into one or more latex baths. The resulting latex layer(s) are then typically leached in water to extract a large percentage of the water-soluble impurities in the latex and coagulant. The coated former is then dried to cure (i.e., crosslink) the rubber. It should be understood that the conditions, process, and materials used in forming natural rubber gloves are well known in the art, and are not critical to the practice of the present invention.

Regardless of the particular material used to form the substrate body 24, the glove 20 includes a hydrogel coating 26 that is present on an inner surface 28 defined by the substrate body 24. The hydrogel coating 26 has a low coefficient of friction that facilitates donning of the glove 20 when the user's hand is either dry or wet, i.e., dry or damp donning. The low coefficient of friction may be imparted through surface texture and/or through the lubricity of the materials used to form the hydrogel coating 26. In addition to facilitating donning, another purpose of the hydrogel coating 26 is to allow stripping of the glove from a former without the use of an antiblocking powder. Specifically, the hydrogel coating 26 blocks the surface of the tacky substrate body 24, thereby preventing it from sticking to itself. Consequently, the glove 20 may be stripped from a former without fear of sticking of the substrate body 24.

Generally speaking, any of a variety of polymers may be utilized in the present invention to form the hydrogel coating 26. Such polymers are formed from at least one hydrogel-forming monomer that is hydrophilic and water-soluble. There are many known hydrophilic, water-soluble monomers that may be used in the present invention to form the hydrogel polymer. Some examples of such monomers include, but are not limited to, vinyl pyrrolidone, hydroxyethyl acrylate or methacrylate (e.g., 2-hydroxyethyl methacrylate), hydroxypropyl acrylate or methacrylate, acrylic or methacrylic acid, acrylic or methacrylic esters or vinyl pyridine, acrylamide, vinyl alcohol, ethylene oxide, derivatives thereof, and so forth. Other examples of suitable monomers are described in U.S. Patent Nos. 4,499,154 to James, et al. The resulting polymers may be homopolymers or interpolymers (e.g., copolymer, terpolymer, etc.), and may be nonionic, anionic, cationic, or amphoteric. In addition, the polymer may be of one type (i.e., homogeneous), or mixtures of different polymers may be used (i.e., heterogeneous).

To form the hydrogel coating 26, the polymer(s) are crosslinked using any known crosslinking technique, including known ionic or covalent crosslinking techniques. For example, in some embodiments, a crosslinking agent may be utilized to facilitate crosslinking. Examples of crosslinking agents include, but are not limited to, polyhydric alcohols (e.g., glycerol); polyaziridine compounds (e.g., 2,2-bishydroxymethyl butanoltris[3-(1-aziridine) propionate] or triaziridine); epoxy compounds; haloepoxy compounds (e.g., epicholorhydrin); aldehyde compounds (e.g., urea-formaldehyde, melamine-formaldehyde, hydantoin-formaldehyde, glutaraldehyde, glyoxal, malonaldehyde, succinaldehyde, adipaldehyde, or dialdehyde starch); polyisocyanate compounds (e.g., 2,4-toluene diisocyanate); etc. Crosslinking may be carried out before, during, and/or after application of the polymer to the surface 28 of the substrate body 24. For example, in one embodiment, an aqueous solution containing a crosslinking agent and polymer is applied to the surface 28. Thereafter, the mixture is cured at elevated temperatures. Besides thermal activation, crosslinking may also be carried out using other well-known techniques. For example, crosslinking may be induced with ionizing radiation, which is radiation having an energy sufficient to either directly or indirectly produce ions in a medium. Some suitable examples of ionizing radiation that may be used in the present invention include, but are not limited to, electron beam radiation, natural and artificial radio isotopes (e.g., $\alpha$, $\beta$, and y rays), x-rays, neutron beams, positively charged beams, laser beams, and so forth. Electron beam radiation, for instance, involves the production of accelerated electrons by an electron beam device. Electron beam devices are generally well known in the art. For instance, in one embodiment, an electron beam device may be used that is available from Energy Sciences, Inc., of Woburn, Massachusetts under the name "Microbeam LV." Other examples of suitable electron beam devices are described in U.S. Patent Nos. 5,003,178 to Livesay; 5,962,995 to Avnery; 6,407,492 to Avnery, et al.

Regardless of the technique utilized, crosslinking forms a hydrogel constituted by a three-dimensional network that is substantially water-insoluble. Thus, when exposed to water, the hydrogel does not dissolve, but instead may absorb a certain amount of water. For example, the hydrogel is capable of achieving a water content of from about 20% to about 90%, in some embodiments from about 35% to about 85%, and in some embodiments, from about 50% to about 80%. The water content of the hydrogel is determined as follows:

$$\% \text{ water} = 100 \times \frac{(\text{weight of wet hydrogel} - \text{weight of dry hydrogel})}{(\text{weight of wet hydrogel})}$$

Besides facilitating donning and allowing stripping of the glove 20 without an antiblocking powder, the hydrogel coating 26 also provides other unexpected benefits. For instance, upon absorbing water, the hydrogel coating 26 swells, thereby increasing the area between crosslinks to form pores. For example, at its highest water content, the hydrogel coating 26 may possess pores having an average size of from about 1 nanometer to about 10 microns, in some embodiments from about 10 nanometers to about 1 micron, and in some embodiments, from about 50 nanometers to about 100 nanometers.

Due to it ability to swell in the above-described manner, an active agent may be incorporated into the hydrogel coating 26 that is controllably releasable therefrom to impart some benefit to a user 22. Specifically, the expected conditions of use expose the hydrogel coating 26 to moisture from a variety of sources, such as water present on a user's hand from washing, moisture secreted by mammalian sweat glands, and so forth. For instance, human sweat glands are of two types, eccrine and apocrine. The apocrine glands occur only in the armpits and about the ears, nipples, navel, and anogenital region, are scent glands. Eccrine glands, however, are present throughout the body, including the hands, and are designed to regulate the temperature of the body. Obviously, the amount of fluid secreted by the eccrine glands depends on body temperature; however, even on cold days, some transepidermal water loss will likely occur. Because elastomeric gloves (e.g., surgical gloves) often fit tightly over a user's hand and do not allow outside air to readily cool the skin, the temperature of the user's hand is likely to increase when wearing the glove. This temperature increase may also cause additional fluid to be secreted by the eccrine glands.

Thus, when placed adjacent to a user's skin, the hydrogel coating 26 will invariably be exposed to fluids secreted by eccrine glands or from some other source. This exposure leads to an increase degree of hydration for the hydrogel coating 26 and a corresponding increase in the size of its pores. As the pore size increases, an active agent within the crosslinked hydrogel network may be released. Once released, the active agent may interact directly with epithelial tissue at the cellular level to provide a benefit to the skin. Alternatively, the active agent may interact with components at or near the skin surface to provide the desired benefit.

The active agent may be incorporated into the hydrogel coating 26 before, during, and/or after its formation. In one embodiment, for example, the active agent may be mixed with the hydrogel-forming polymer and crosslinking agent prior to crosslinking. When crosslinked, the active agent is retained within the three-dimensional network. As stated, the active agent may also be applied after formation of the hydrogel. For example, the hydrogel coating 26 may be applied with an aqueous solution containing the active agent. As described above, the aqueous solution hydrates the hydrogel coating 26 and causes an increase in porosity. Due to this increased porosity, the active agent may diffuse through the pores and into the crosslinked hydrogel network. The hydrogel coating 26 is subsequently dried to retain the active agent therein. Typically, the size of the active agent is smaller than the pore size of the hydrogel when dry so that it remains physically retained within the hydrogel network. Apart from being physically retained within the hydrogel coating 26, the active agent may also be chemically bonded to the hydrogel, such as through covalent, ionic, or hydrogen bonding.

Generally speaking, the "active agent" may be any compound or mixture thereof that may produce a desired result. Whether in solid or liquid form, the active agent typically possesses a sufficient solubility or miscibility in an aqueous system to render it capable of being released through the pores of the crosslinked hydrogel network. Examples of such active agents include, but are not limited to, drugs, skin-conditioners, botanical agents, etc. "Drugs" include any physiologically or pharmacologically active substance that produces a localized or a systemic effect in animals. The drugs that may be delivered include, but are not limited to, anti-inflammatory agents, immunosuppressives, antimicrobials, anesthetics, analgesics, hormones, antihistamines, and so forth. Numerous such compounds are known to those of skill in the art and described, for example, in The Pharmacological Basis of Therapeutics, Hardman, Limbird, Goodman & Gilman, McGraw-Hill, New York, (1996), as well as U.S. Patent Nos. 6,419,913 to Niemiec, et al.; 6,562,363 to Mantelle, et al.; 6,593,292 to Rothbard, et al.; 6,567,693 to Allen, Jr.; and 6,645,181 to Lavi, et al. Although several examples of active agents are described herein, it should be understood that the present invention is by no means limited to any particular active agent. In fact, any active agent having any benefit whatsoever to a user may be utilized in accordance with the present invention.

In this regard, one class of suitable drugs includes anti-inflammatory agents, such as glucocorticoids (adrenocorticoid steroids). Exemplary glucocorticoids include, for example, hydrocortisone, prenisone (deltasone) and predrisonlone (hydeltasol). Glucocorticoids may be used to treat inflammatory skin diseases, such as eczema (e.g., atopic dermatitis, contact dermatitis, and allergic dermatitis), bullous disease, collagen vascular diseases, sarcoidosis, Sweet's disease, pyoderma gangrenosum, Type I reactive leprosy, capillary hemangiomas, lichen planus, exfoliative dermatitis, erythema nodosum, hormonal abnormalities (including acne and hirsutism), toxic epidermal necrolysis, erythema multiforme, cutaneous T-cell lymphoma, discoid lupus erythematosus, and so forth. Retinoids, such as retinol, tretinoin, isotretinoin, etretinate, acitretin, and arotinoid, may also be used. Conditions that are possibly treatable using retinoids include, but are not limited to, acne, keratinization disorders, psoriasis, cutaneous aging, discoid lupus erythematosus, scleromyxedema, verrucous epidermal nevus, subcorneal pustular dermatosis, Reiter's syndrome, warts, lichen planus, acanthosis nigricans, sarcoidosis, Grover's disease, porokeratosis, and so forth. Other suitable anti-inflammatory drugs are COX-2 inhibitors, such as celecoxib, meloxicam, rofecoxib, and flosulide. These drugs inhibit the production of the COX-2 (cyclooxygenase-2) enzyme induced by pro-inflammatory stimuli in migratory cells and inflamed tissue. In addition, nonsteroidal anti-inflammatory drugs (NSAIDs) may also be utilized. Examples of NSAIDs include, but are not limited to, Aspirin, Ibuprofen, Indomethacin, Phenylbutazone, Bromfenac, Sulindac, Nabumetone, Ketorolac, Mefenamic Acid, and Naproxen.

Immunosuppressive drugs constitute an additional class of drugs from which the active agent may be selected. These drugs may be used to treat hyperproliferative diseases, such as psoriasis, as well as immune diseases, such as bullous dermatoses and leukocytoclastic vasculitis. Examples of such drugs include, but are not limited to, antimetabolites, such as methotrexate, azathioprine, fluorouracil, hydroxyurea, 6-thioquanine, mycophenolate, chlorambucil, vinicristine, vinblasrine and dactinomycin; alkylating agents, such as cyclophosphamide, mechloroethamine hydrochloride, carmustine, taxol, tacrolimus and vinblastine; and so forth.

Another class of suitable drugs includes antimicrobial agents, e.g., antibacterial, antifungal, antiviral, etc. Antibacterial agents are useful for treating conditions such as acne, cutaneous infections, and so forth. For instance, some suitable antimicrobial agents include, but are not limited to, bisphenols, such as 2,4,4'-trichloro-2'-hydroxydiphenyl ether (triclosan); quaternary ammonium compounds, such as benzalkonium chloride; esters of parahydroxy benzoic acid, such as methyl parabens; formaldehyde and formaldehyde donors, such as 2-bromo-2-nitro-1,3 propanediol, hydantoins, diazolidinyl urea, and imidazolidinyl urea; alkylisothizaolinones; phenoxyethanol; chlorhexidine gluconate; parachlorometaxylenol (PCMX); chitosan, such as chitosan pyrrolidone carboxylate; combinations thereof, and so forth. Antifungal agents may also be used to treat conditions, such as tinea corporis, tinea pedis, onychomycosis, candidiasis, tinea versicolor, onychomycosis, and so forth. Examples of antifungal agents include, but are not limited to, azole antifungals such as itraconazole, myconazole and fluconazole. Examples of antiviral agents include, but are not limited to, acyclovir, famciclovir, and valacyclovir. Such agents are useful for treating viral diseases, such as herpes.

Antihistamines are still another class of suitable drugs. Examples of such antihistamines include, for example, terfenadine, astemizole, lorotadine, cetirizine, acrivastine, temelastine, cimetidine, ranitidine, famotidine, nizatidine, and so forth. These agents may be used to treat conditions such as pruritus, atopic dermatitis, contact dermatitis, psoriasis, etc. Further, local anesthetics constitute another class of drugs that may be used. Examples of such local anesthetics include, but are not limited to, lidocaine, bupibacaine, novocaine, procaine, tetracaine, benzocaine, mepivacaine, etidocaine, 2-chloroprocaine hydrochloride, and so forth.

Other than drugs, various other active agents may be released from the glove according to the present invention. For instance, in some embodiments, the active agent may be a skin-conditioner that improves moisture retention, softness, texture, and other properties of the skin. One example of such a skin-conditioner is an emollient that helps restore dry skin to a more normal moisture balance. Specifically, emollients act on the skin by supplying fats and oils that blend with skin, making it pliable, repairing some of the cracks and fissures in the stratum corneum, and forming a protective film that traps water in the skin. Emollients that may be suitable for use in the present invention include, but are not limited to, beeswax, butyl stearate, cermides, cetyl palmitate, eucerit, isohexadecane, isopropyl palmitate, isopropyl myristate, mink oil, mineral oil, nut oil, oleyl alcohol, petroleum jelly or petrolatum, glyceral stearate, avocado oil, jojoba oil, lanolin (or woolwax), lanolin derivatives such as lanolin alcohol, retinyl palmitate (a vitamin A derivative), cetearyl alcohol, squalane, squalene, stearic acid, stearyl alcohol, myristal myristate, various lipids, decyl oleate and castor oil. Another possible skin conditioner is a humectant, which may supply the skin with water by attracting moisture from the air and holding it on the skin. Humectants that may be suitable in the present invention include, but are not limited to, alanine, glycerin, polyethylene glycol, propylene glycol, butylene glycol, hyaluronic acid, Natural Moisturizing Factor (a mixture of amino acids and salts that are among the skin's natural humectants), saccharide isomerate, sodium lactate, sorbitol, urea, and so forth. Still other suitable skin-conditioners include antioxidants, a group of substances that prevent or slow the oxidation process of skin, thereby protecting it from premature aging. Exemplary antioxidants include, but are not limited to, Vitamin E, Vitamin E derivatives, Vitamin C, Vitamin C derivatives, Vitamin A palmitate, butylated hydroxy toluene, phenols, phenolic derivatives, thiodipropionate esters, hydroquinone derivatives, alkylated aryl amine,

5

combinations thereof, and so forth.

The active agent may also be a botanical agent that may potentially reduce swelling, itching, reddening, etc. Examples of some botanicals that may be used include, but are not limited to, Agnus castus, aloe vera, comfrey, calendula, dong quai, black cohosh, chamomile, evening primrose, Hypericum perforatum, licorice root, black currant seed oil, St. John's wort, tea extracts, lemon balm, capsicum, rosemary, Areca catechu, mung bean, borage seed oil, witch hazel, fenugreek, lavender, soy, almonds, chamomile extracts (e.g., bisabolol), elder flowers, honey, safflower oil, and elastin.

The hydrogel coating 26 may be applied to the substrate body 24 using any suitable method. For example, techniques, such.as dipping, spraying, patting, tumbling, etc., may be utilized in the present invention. The average thickness of the hydrogel coating 26, when dry, may range from about 0.05 to about 50 micrometers, in some embodiments from about 0.1 to about 20 micrometers, and in some embodiments, from about 1 to about 10 micrometers. Such a thin coating may provide enhanced donning benefits to the glove 20. In addition, the dried hydrogel coating 26 may comprise from about 0.0001 to about 1 gram per gram of the glove, in some embodiments from about 0.001 to about 0.5 grams per gram of the glove, and in some embodiments, from about 0.01 to about 0.2 grams per gram of the glove 20.

A lubricant coating 32 averties the hydrogel coating 26 to aid in damp donning. The lubricant coating 32 may cover only a portion of the hydrogel coating 26, or cover its entire surface. In the illustrated embodiment, for example, the lubricant coating 32 covers the entire surface of the hydrogel coating 26 and defines a wearer-contacting surface 27 of the glove 20 that contacts the body of the wearer 22 during use. Alternatively, however, the lubricant coating 32 may cover only those portions of the substrate body 30 not already covered by the hydrogel coating 26.

The lubricant coating 32 comprises any suitable silicone emulsion, such as those described in U.S. Patent Application Publication No. 2003/0118837 to Modha, et al. The solids content of the silicone emulsion may be from about 0.1 wt.% to about 10 wt.%, in some embodiments from about 0.25 wt.% to about 5 weight %, and in some embodiments, from about 0.3 wt.% to about 1 wt.%. To lower the solids content of a commercially available silicone emulsion, for example, additional amounts of solvent may be utilized. By varying the solids content, the presence of silicone in the glove 20 may be controlled. For example, to form a glove with a higher degree of donning properties, the silicone emulsion may have a relatively high solids content so that a greater percentage of the silicone is incorporated into the coating 32 during the forming process. The thickness of the lubricant coating 32 may also vary. For example, the thickness,may range from about 0.001 millimeters to about 0.4 millimeters. In another embodiment, the thickness may range from about 0.01 millimeters to about 0.30 millimeters. In still another embodiment, the thickness may range from about 0.01 millimeters to about 0.20 millimeters.

In one particular embodiment, the silicone emulsion used to form the lubricant coating 32 is DC 365, which is a pre-emulsified silicone (35% solids content) that is commercially available from Dow Corning Corporation (Midland, Michigan) and believed to contain 40-70% water (aqueous solvent), 30-60% methyl- modified polydimethylsiloxane (silicone), 1-5% propylene glycol (nonaqueous solvent), 1-5% polyethylene glycol sorbitan monolaurate'(nonionic surfactant), and 1-5% octylphenoxy polyethoxy ethanol (nonionic surfactant). In another embodiment, the silicone emulsion is SM 2140 (50% solids content), which is a pre-emulsified silicone that is commercially available from GE Silicones (Waterford, New York) and believed to contain 30-60% water (aqueous solvent), 30-60% amino-modified dimethylpolysiloxane (silicone), 1-5% ethoxylated nonyl phenol (nonionic surfactant), 1-5% trimethyl-4-nonyloxypolyethyleneoxy ethanol (nonionic surfactant), and minor percentages of various other components. In still another embodiment, the silicone emulsion is SM 2150 (50% solids content), which is a pre-emulsified silicone that is commercially available from GE Silicones (Waterford, New York) and believed to contain 30-60% water (aqueous solvent), 30-60% amino-modified dimethylpolysiloxane (silicone), 1-5% polyoxyethylene lauryl ether, 1-5% a nonionic surfactant, and minor percentages of various other components. If desired, these pre-emulsified silicones may be diluted with water or other solvents prior to use in the lubricant coating 32.

The lubricant coating 32 may also include a cationic surfactant (e.g., cetyl pyridnium chloride), an anionic surfactant (e.g., sodium lauryl sulfate), or a nonionic surfactant. For instance, in one embodiment, the lubricant coating 32 contains a quaternary ammonium compound, such as Varisoft BTMS (available from Goldschmidt Chemical Corp. of Dublin, Ohio) and a silicone emulsion (AF-60) obtained from General Electric Silicone. Varisoft BTMS contains behnyl trimethyl sulfate and cetyl alcohol, while AF-60 contains polydimethylsiloxane, acetylaldehyde, and small percentages of emulsifiers. Various other suitable lubricants are described, for instance, in U.S. Patent Nos. 5,742,943 to Chen and 6,306,514 to Weikel, et al.

Further, besides the above-mentioned layers, the glove 20 may also contain additional layers if desired. For example, in one embodiment, the gripping surface 30 of the glove 20 contains a silicone emulsion, such as described above, that improves the gripping characteristics of the glove 20 by inhibiting chlorination of the surface 30.

An elastomeric article made in accordance with the present invention may generally be formed using a variety of processes known in the art. In fact, any process capable of making an elastomeric article may be utilized in the present invention. For example, elastomeric article formation techniques may utilize dipping, spraying, chlorination, drying, curing, as well as any other technique known in the art. In this regard, one embodiment of an "on-line" method of dip forming a glove formed from natural rubber latex will now be described in more detail.

Initially, any well-known former, such as formers made from metals, ceramics, or plastics, is provided. Although glove-shaped formers are described herein, it should also be understood that formers having any other shape (e.g., condom-shaped) may be used in accordance with the present invention to form articles having different shapes. The former is dipped into a bath containing a coagulant, a surfactant, water, and optionally other ingredients, such as a salt that contains calcium ions (e.g., calcium nitrate and/or calcium carbonate) to break the protection system of a natural rubber latex emulsion. The salt may also facilitate removal of the tacky latex from the former, thus acting as a release agent. The surfactant provides good wetting to avoid forming a meniscus and trapping air between the form and deposited latex, particularly in the cuff area. If desired, the former may be preheated so that the residual heat dries off the water leaving, for example, calcium nitrate, calcium carbonate, and surfactant on the surface of the former. Other suitable coagulant solutions are also described in U.S. Patent No. 4,310,928 to Joung

After being immersed in the coagulant composition, the former is withdrawn and allowed to dry. The former is then dipped into a tank containing a natural rubber latex bath to form the substrate body 24. The bath contains, for example, natural rubber latex, stabilizers, antioxidants, curing activators, organic accelerators, vulcanizers, and so forth. The stabilizers may, for example, be phosphate-type surfactants. The antioxidants may be phenol-based compounds, such as 2,2'-methylenebis (4-methyl-6-t-butylphenol). The curing activator may be zinc oxide. The organic accelerator may be dithiocarbamate. The vulcanizer may be sulfur or a sulfur-containing compound. If these materials are used, the stabilizer, antioxidant, activator, accelerator and vulcanizer may be dispersed into water to avoid crumb formation by using a ball mill. This dispersion is then mixed into the latex. The former is dipped into one or more latex baths a sufficient number of times to build up the desired thickness on the former. By way of example, the substrate body 24 may have a thickness of from about 0.1 to about 0.3 millimeters. A bead roll station may, in some embodiments, be utilized to impart a cuff to the glove 20. The latex-coated former is then dipped into a leaching tank in which hot water is circulated to remove the water-soluble components, such as residual calcium nitrates and proteins contained in the natural latex. This leaching process may continue for about twelve minutes with the tank water being about 49°C.

The coated former may then be dipped one or more times into a solution to form the hydrogel coating 26 of the glove 20. In one embodiment, the former is dipped into an aqueous solution of a water-soluble hydrogel-forming polymer or a mixture of such polymers. The aqueous composition may, for instance, include from about 0.1 wt.% to about 30 wt.% of hydrogel-forming polymer(s), in some embodiments from about 0.5 wt.% to about 10 wt.% of hydrogel-forming polymer(s), and in some embodiments, from about 1 wt.% to about 5 wt.% of hydrogel-forming polymer(s). The aqueous solution may also contain from about 0.01 wt.% to about 10 wt.% crosslinking agent(s), in some embodiments from about 0.1 wt.% to about 5 wt.% crosslinking agent(s), and in some embodiments, from about 0.2 wt.% to about 2 wt.% crosslinking agent(s). Water typically constitutes from about 70 wt.% to about 99.9 wt.%, and in some embodiments, from about 90 wt.% to about 99 wt.% of the aqueous solution.

The aqueous solution may also contain other components. For example, the solution may also contain an active agent that is releasable from the hydrogel coating 26. The amount of the active employed may vary depending on the type of active agent, the type of hydrogel, etc. Specifically, hydrogels that provide a slow release rate may require a higher active agent content than hydrogels that provide a fast release rate. For example, the aqueous solution may contain from about 0.0001 wt.% to about 30 wt.% of active agent(s), in some embodiments from about 0.001 wt.% to about 10 wt.% active agent(s), and in some embodiments, from about 0.1 wt.% to about 5 wt.% active agent(s). In addition, to initiate or speed up the crosslinking process, a catalyst, such as p-toluene sulfonic acid or hydrochloric acid, may be utilized. Polymerization initiators may also be utilized, such as described in U.S. Patent No. 6,242,042 to Goldstein, et al. A pH adjuster, such as an acid or base, may be also be added to achieve a certain pH.

Once coated, the former is sent to a curing station (e.g., oven) where the natural rubber is vulcanized and the hydrogel-forming polymer is crosslinked. If desired, the curing station may initially evaporate any remaining water and then proceed to the higher temperature vulcanization and crosslinking steps. For instance, curing of the hydrogel-forming polymer may be initiated by heating at a temperature from about 25°C to about 200°C, in some embodiments from about 50°C to about 150°C, and in some embodiments from about 70°C to about 120°C, for a period of time of from about 1 minute to about 12 hours, in some embodiments from about 5 minutes to about 5 hours, and In some embodiments, from about 10 minutes to about 1 hour. Vulcanization may occur at the same time as the crosslinking of the hydrogel-forming polymer, or at a different time. If desired, the oven may be divided into four different zones with a former being conveyed through the zones of increasing temperature. One example is an oven having four zones with the first two zones being dedicated to drying, and the second two zones being primarily to vulcanization and crosslinking of the hydrogel polymer. Each of the zones may have a slightly higher temperature, for example, the first zone at about 80°C, the second zone at about 95°C, a third zone at about 105°C, and a final zone at about 115°C. The residence time of the former within a zone may, for instance, be about 10 minutes.

Regardless of the technique used to form the glove 20, it has been discovered that various treatment steps conventionally conducted "off-line" (i.e., after stripping) may be conducted "on-line" in accordance with the present invention. For example, the lubricant coating 32 is applied to the glove 20 while it is still present on the former. In one particular embodiment, a silicone emulsion is first thoroughly mixed with water using a high shear mixer to achieve a homogeneous solution having the desired solids content. Thereafter, the resulting emulsion is then applied to in a variety of ways without removing the glove 20 from the former. For instance, the silicone emulsion may be sprayed onto the glove 20 using a conventional spray nozzle. Alternatively, the glove 20 may be dipped into a silicone emulsion to form the lubricant coating 32. Once applied with the silicone emulsion, the silicone-coated glove is then dried. For example, in some embodiments, the silicone-coated glove may be dried at a temperature of from about 20°C to about 200°C, in some embodiments from about 30°C to about 150°C, and in some embodiments, from about 35°C to about 115°C. It should be understood that the desired drying temperature may vary widely depending on the polymer(s) used to form the substrate body 24.

In addition to being applied with the lubricant coating 32, the glove 20 may also be halogenated "on-line" while still present on the former. For example, chlorination may be performed by immersing the glove 20 in an aqueous solution containing dissolved chlorine. The concentration of chlorine may be monitored and controlled to control the degree of chlorination. The chlorine concentration is typically at least about 100 parts per million (ppm), in some embodiments from about 200 ppm to about 3500 ppm, and in some embodiments, from about 300 ppm to about 600 ppm, e.g., about 400 ppm. The time duration of the chlorination step may also be controlled to control the degree of chlorination and may range, for example, from about 1 to about 10 minutes, e.g., 4 minutes. The glove 20 may then be rinsed with tap water at about room temperature. This rinse cycle may be repeated as necessary. Once all water is removed, the glove 20 may be dried to remove excess water. Other chlorination techniques are described in U.S. Patent Nos. 3,411,982 to Kavalir; 3,740,262 to Agostinelli; 3,992,221 to Homsy, et al.; 4,597,108 to Momose; and 4,851,266 to Momose, 5,792,531 to Littleton, et al..

Thereafter, the glove 20 may be stripped from the former and turned inside out. The hydrogel coating 26 inhibits the tacky substrate body 24 from sticking to itself. Once turned inside out and removed the former, the hydrogel coating 26 and the optional lubricant coating 32 form the wearer-contacting surface 27 of the glove 20. Although often undesired, the glove 20 may optionally be subjected to various "off-line" treatments, such as chlorination and/or lubrication.

The present invention may be better understood with reference to the following example.

### EXAMPLE

The ability to form an elastomeric article in accordance with the present invention was demonstrated. Initially, a glove-shaped former was dipped into a dip tank that contained a coagulant composition. Specifically, the coagulant composition contained 15% by weight calcium nitrate, 6 % by weight calcium carbonate, 0.15% surfactant, and water so that the resulting solids content was about 21 %. After dipping, the former into the coagulant, it was removed from the coagulant composition and dried a temperature of 115°C. Next, the former was dipped into a compound of natural rubber latex and allowed to air dry. The resulting substrate body was beaded and leached with water. The thickness of the resulting substrate body was 0.27 millimeters.

The glove was then dipped for 5 to 10 seconds in a polyalcohol-based primer (minimum solids content of 20%) available from Delta Polymer Systems Sdn. Bhd*. of Selangor, Malaysia under the name "ACTIVE BOND." After drying the primer, the glove was dipped for 5 to 10 seconds into a sequentially mixed solution of water, phosphoric acid, "BYOSYLK", "BYOSYLK" Part B, and "BYOSYLK" Part A. "BYOSYLK" is polyacrylate-based and has a minimum solids content of 10%, while "BYOSYLK" Part B and "BYOSYLK" Part A are polyalcohol-based and have a minimum solids content of 80% and 11 %, respectively. "BYOSYLK", "BYOSYLK" Part B, and "BYOSYLK" Part A are each available from Delta Polymer Systems Sdn. Bhd. of Selangor, Malaysia. The thickness of the resulting hydrogel coating was 2 to 4 micrometers.

After being applied with the hydrogel-forming solution, the glove entered a second beading station and was placed in an oven at 130°C to 150°C for 40 to 60 minutes, wherein the hydrogel polymer and the natural rubber polymer composition were cured. The coated former was cooled and dipped into a solution of chlorine (concentration of 50 to 200 ppm) for 5 to 10 seconds to chlorinate the hydrogel. The coated former was then dipped into a tank of tap water, and immersed for 5 to 10 seconds into a silicone emulsion having a solids content of from 0.3 to 1.0 wt.%. The coating was subsequently dried at 100°C to 120° C, and then stripped from the former.

While the invention has been described in detail with respect to the specific embodiments thereof, it will be appreciated that those skilled in the art, upon attaining an understanding of the foregoing, may readily conceive of alterations to, variations of, and equivalents to these embodiments. Accordingly, the scope of the present invention should be assessed as that of the appended claims

**Claims**

1. A method for forming an elastomeric glove, said method comprising:

dipping a hand-shaped former into at least one bath containing an elastomeric material to form a substrate body, said substrate body having an inner surface and an outer surface that define a hand-shaped cavity, said inner surface being positioned adjacent to said hand-shaped former;

applying a hydrogel coating to said outer surface of said substrate body while said inner surface of said substrate body remains adjacent to said hand-shaped former, wherein said hydrogel coating has a thickness of from about 0.1 to about 20 micrometers;

applying a lubricant coating to said hydrogel-coated substrate body while the inner surface of the substrate body remains adjacent to the hand-shaped former, wherein said lubricant coating comprises a silicone emulsion; and

thereafter, stripping the glove from said hand-shaped former without the use of an antiblocking powder, wherein the glove is inverted so that said outer surface of said substrate body applied with said hydrogel coating is configured to face a user's hand when inserted into said hand-shaped cavity.

2. A method as defined in claim 1, wherein said hydrogel coating is formed by crosslinking a hydrogel-forming polymer to form a substantially water-insoluble hydrogel network.

3. A method as defined in claim 2, wherein said hydrogel-forming polymer is formed from at least one monomer that is hydrophilic and water-soluble.

4. A method as defined in claim 3, wherein said monomer is selected from the group consisting of vinyl pyrrolidones, hydroxyethyl acrylates, hydroxyethyl methacrylates, hydroxypropyl acrylates, hydroxypropyl methacrylates, acrylic acids, methacrylic acids, acrylic esters, methacrylic esters, vinyl pyridines, acrylamides, vinyl alcohols, ethylene oxides, derivatives thereof, and combinations thereof.

5. A method as defined in claim 3, wherein said monomer is selected from the group consisting of hydroxyethyl acrylates, hydroxyethyl methacrylates, hydroxypropyl acrylates, derivatives thereof, and combinations thereof.

6. A method as defined in any of the preceding claims, wherein said elastomeric material of said substrate body includes an emulsion-based elastomeric material.

7. A method as defined in claim 6, wherein said emulsion-based elastomeric material is selected from the group consisting of natural rubber latex, Isoprene polymers, chloroprene polymers, vinyl chloride polymers, butadiene polymers, styrene-butadiene polymers, carboxylated styrene-butadiene polymers, acrylonitrile-butadiene polymers, carboxylated acrylonitrile-butadiene polymers, acrylonitrile-styrene-butadiene polymers, carboxylated acrylonitrile-styrene-butadiene polymers, derivatives thereof, and combinations thereof.

8. A method as defined in claim 6, wherein said elastomeric material of said substrate body includes natural rubber latex.

9. A method as defined in any preceding claim, wherein said lubricant further comprises a surfactant.

10. A method as defined in any of the preceding claims, wherein said hydrogel coating further contains an active agent capable of imparting a benefit to a user.

11. A method as defined in any of the preceding claims, further comprising chlorinating the glove prior to stripping the glove from said hand-shaped former.


**Patentansprüche**

1. Verfahren zum Bilden eines elastomeren Handschuhs, wobei das Verfahren umfasst:

Eintauchen eines handförmigen Formers in wenigstens ein Bad, das ein elastomeres Material enthält, zum Bilden eines Substratkörpers, wobei der Substratkörper eine innere Oberfläche und eine äußere Oberfläche hat, die einen handförmigen Hohlraum definieren, wobei die innere Oberfläche an den handförmigen Former anliegend positioniert ist,

Aufbringen einer Hydrogelbeschichtung auf die äußere Oberfläche des Substratkörpers, während die innere Oberfläche des Substratkörpers an dem handförmigen Former anliegend verbleibt, worin die Hydrogelbeschichtung eine Dicke von etwa 0,1 bis etwa 20 Mikrometer hat,

Aufbringen einer Gleitmittelbeschichtung auf den hydrogelbeschichteten Substratkörper, während die innere Oberfläche des Substratkörpers auf dem handförmigen Former anliegend verbleibt, worin die Gleitmittelbeschichtung eine Siliconemulsion umfasst, und

danach Abstreifen des Handschuhs von dem handförmigen Former ohne Verwendung eines Antiblockierpulvers, wobei der Handschuh invertiert wird, so dass die äußere Oberfläche des Substratkörpers mit der aufgebrachten Hydrogelbeschichtung konfiguriert wird, um sich der Hand eines Benutzers gegenüber zu befinden, wenn diese in den handförmigen Hohlraum eingeführt wird.

**2.** Verfahren wie in Anspruch 1 definiert, worin die Hydrogelbeschichtung durch Vernetzen eines hydrogelbildenden Polymers zum Bilden eines im Wesentlichen wasserunlöslichen Hydrogelnetzwerkes gebildet wird.

**3.** Verfahren wie in Anspruch 2 definiert, worin das hydrogelbildende Polymer aus wenigstens einem Monomer gebildet wird, das hydrophil und wasserlöslich ist.

**4.** Verfahren wie in Anspruch 3 definiert, worin das Monomer ausgewählt ist aus der Gruppe bestehend aus Vinylpyrrolidonen, Hydroxyethylacrylaten, Hydroxyethylmethacrylaten, Hydroxypropylacrylaten, Hydroxypropylmethacrylaten, Acrylsäuren, Methacrylsäuren, Acrylestern, Methacrylestern, Vinylpyridinen, Acrylamiden, Vinylalkoholen, Ethylenoxiden, Derivaten davon und Kombinationen davon.

**5.** Verfahren wie in Anspruch 3 definiert, worin das Monomer ausgewählt ist aus der Gruppe bestehend aus Hydroxyethylacrylaten, Hydroxyethylmethacrylaten, Hydroxypropylacrylaten, Derivaten davon und Kombinationen davon.

**6.** Verfahren wie in einem der vorhergehenden Ansprüche definiert, worin das elastomere Material des Substratkörpers ein elastomeres Material auf Emulsionsbasis enthält.

**7.** Verfahren wie in Anspruch 6 definiert, worin das elastomere Material auf Emulsionsbasis ausgewählt ist aus der Gruppe bestehend aus Naturkautschuklatex, Isoprenpolymeren, Chloroprenpolymeren, Vinylchloridpolymeren, Butadienpolymeren, Styrol-Butadien-Polymeren, carboxylierten Styrol-Butadien-Polymeren, Acrylnitril-Butadien-Polymeren, carboxylierten Acrylnitril-Butadien-Polymeren, Acrylnitril-Styrol-Butadien-Polymeren, carboxylierten Acrylnitril-Styrol-Butadien-Polymeren, Derivaten davon und Kombinationen davon.

**8.** Verfahren wie in Anspruch 6 definiert, wobei das elastomere Material des Substratkörpers Naturkautschuklatex enthält.

**9.** Verfahren wie in einem der vorhergehenden Ansprüche definiert, worin das Gleitmittel weiter einen oberflächenaktiven Stoff enthält.

**10.** Verfahren wie in einem der vorhergehenden Ansprüche definiert, worin die Hydrogelbeschichtung weiter ein aktives Agens enthält, das in der Lage ist, einem Benutzer einen Vorteil zu verleihen.

**11.** Verfahren wie in einem der vorhergehenden Ansprüche definiert, das weiter das Chlorieren des Handschuhs vor dem Abstreifen des Handschuhs von dem handförmigen Formen umfasst.

**Revendications**

**1.** Procédé de formation d'un gant élastomère, ledit procédé comprenant :

le trempage d'un formeur en forme de main dans au moins un bain contenant un matériau élastomère pour former un corps substrat, ledit corps substrat ayant une surface interne et une surface externe qui définissent une cavité en forme de main, ladite surface interne étant positionnée adjacente audit formeur en forme de main ;
l'application d'un enrobage d'hydrogel à ladite surface externe dudit corps substrat tandis que ladite surface interne dudit corps substrat reste adjacente audit formeur en forme de main, ledit enrobage d'hydrogel ayant une épaisseur comprise entre environ 0,1 et environ 20 micromètres ;
l'application d'un enrobage lubrifiant audit corps substrat enrobé d'hydrogel tandis que la surface interne du corps substrat reste adjacente au formeur en forme de main, ledit enrobage lubrifiant comprenant une émulsion de silicone ;
puis le fait d'enlever le gant dudit formeur en forme de main sans recours à l'utilisation d'une poudre antiblocage', le gant étant retourné de sorte que ladite surface externe dudit corps substrat sur laquelle est appliqué ledit enrobage d'hydrogel est configurée pour être tournée vers la main d'un utilisateur lorsque cette main est insérée dans ladite cavité en forme de main.

2. Procédé selon la revendication 1, dans lequel ledit enrobage d'hydrogel est formé par réticulation d'un polymère formateur d'hydrogel pour former un réseau d'hydrogel sensiblement hydro-insoluble.

3. Procédé selon la revendication 2, dans lequel ledit polymère formateur d'hydrogel est formé à partir d'au moins un monomère qui est hydrophile et hydrosoluble.

4. Procédé selon la revendication 3, dans lequel ledit monomère est sélectionné dans le groupe consistant en les vinyl pyrrolidones, les acrylates d'hydroxyéthyle, les méthacrylates d'hydroxyéthyle, les acrylates d'hydroxypropyle, les méthacrylates d'hydroxypropyle, les acides acryliques, les acides méthacryliques, les esters acryliques, les esters méthacryliques, les vinyl pyridines, les acrylamides, les alcools vinyliques, les oxydes d'éthylène, leurs dérivés et leurs combinaisons.

5. Procédé selon la revendication 3, dans lequel ledit monomère est sélectionné dans le groupe consistant en les acrylates d'hydroxyéthyle, les méthacrylates d'hydroxyéthyle, les acrylates d'hydroxypropyle, leurs dérivés et leurs combinaisons.

6. Procédé selon l'une quelconque des revendications précédentes, dans lequel ledit matériau élastomère.dudit corps substrat inclut un matériau élastomère à base d'émulsion.

7. Procédé selon la revendication 6, dans lequel ledit matériau élastomère à base d'émulsion est sélectionné dans le groupe consistant en le latex de caoutchouc naturel, les polymères d'isoprène, les polymères de chloroprène, les polymères du chlorure de vinyle, les polymères de butadiène, les polymères de styrène-butadiène, les polymères de styrène-butadiène carboxylés, les polymères d'acrylonitrile-butadiène, les polymères d'acrylonitrilebutadiène carboxylés, les polymères acrylonitrile-styrène-butadiène, les polymères acrylonitrile-styrèrie-butadiène carboxylés, leurs dérivés et leurs combinaisons.

8. Procédé selon la revendication 6, dans lequel ledit matériau élastomère dudit corps substrat inclut un latex de caoutchouc naturel.

9. Procédé selon l'une quelconque des revendications précédentes, dans lequel ledit lubrifiant comprend, en outre, un tensio-actif.

10. Procédé selon l'une quelconque des revendications précédentes, dans lequel ledit enrobage d'hydrogel contient, en outre, un agent actif capable d'offrir un effet bénéfique à un utilisateur.

11. Procédé selon l'une quelconque des revendications précédentes, comprenant, en outre, la chloration du gant avant d'enlever le gant dudit formeur en forme de main.

FIG. 1

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4499154 A
- WO 0232475 A
- US 5003178 A, Livesay
- US 5962995 A, Avnery
- US 6407492 A, Avnery
- US 6419913 B, Niemiec
- US 6562363 B, Mantelle
- US 6593292 B, Rothbard
- US 6567693 B, Allen, Jr.
- US 6645181 B, Lavi
- US 20030118837 A, Modha

- US 5742943 A, Chen
- US 6306514 A, Weikel
- US 4310928 A, Joung
- US 6242042 B, Goldstein
- US 3411982 A, Kavalir
- US 3740262 A, Agostinelli
- US 3992221 A, Homsy
- US 4597108 A, Momose
- US 4851266 A, Momose
- US 5792531 A, Littleton

**Non-patent literature cited in the description**

- **HARDMAN ; LIMBIRD ; GOODMAN ; GILMAN.** The Pharmacological Basis of Therapeutics. Mc-Graw-Hill, 1996